(19) Europäisches Patentamt
European Patent Office
Office européen des brevets

(11) Numéro de publication: **0 587 637 B1**

(12) # FASCICULE DE BREVET EUROPEEN

(45) Date de publication du fascicule du brevet: **16.08.95**

(51) Int. Cl.$^{6}$: **A61K 7/06**

(21) Numéro de dépôt: **92911070.8**

(22) Date de dépôt: **02.06.92**

(86) Numéro de dépôt internationale :
**PCT/FR92/00485**

(87) Numéro de publication internationale :
**WO 92/21316 (10.12.92 92/31)**

(54) **COMPOSITION DE TRAITEMENT DES MATIERES KERATINIOUES A BASE DE SILICONE ET DE LATEX.**

(30) Priorité: **03.06.91 FR 9106657**

(43) Date de publication de la demande:
**23.03.94 Bulletin 94/12**

(45) Mention de la délivrance du brevet:
**16.08.95 Bulletin 95/33**

(84) Etats contractants désignés:
**AT BE CH DE DK ES FR GB GR IT LI NL SE**

(56) Documents cités:
**EP-A- 0 214 626**
**EP-A- 0 266 921**
**EP-A- 0 424 260**
**GB-A- 2 114 580**

(73) Titulaire: **L'OREAL**
**14, rue Royale**
**F-75008 Paris (FR)**

(72) Inventeur: **DUBIEF, Claude**
**9, rue Edmond-Rostand**
**F-78150 Le Chesnay (FR)**
Inventeur: **CAUWET, Danièle**
**53, rue de Charonne**
**F-75011 Paris (FR)**

(74) Mandataire: **Casalonga, Axel**
**BUREAU D.A. CASALONGA - JOSSE**
**Morassistrasse 8**
**D-80469 München (DE)**

**EP 0 587 637 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet une composition de traitement des matières kératiniques, en particulier des cheveux, contenant une silicone, un latex et un agent de mise en suspension du latex et de la silicone et/ou un épaississant, aux procédés de mise en oeuvre et en particulier au traitement cosmétique mettant en oeuvre cette composition.

Il est bien connu que les cheveux sont abimés, fragilisés par l'action des agents atmosphériques extérieurs, tels que la lumière et les intempéries et par des traitements mécaniques ou chimiques, tels que le brossage, le peignage, la chaleur, les déformations permanentes, les colorations ou décolorations.

Dans certains cas, ils sont si abîmés qu'on constate aussi la présence de fourches sur les extrémités des cheveux; ces fourches sont dues à des ruptures longitudinales de la fibre, notamment au niveau des pointes. Les cheveux sont alors rêches et difficiles à démêler.

On est, de ce fait, à la recherche de compositions de traitement des fibres kératiniques permettant de conférer des propriétés de douceur et de démêlage en restructurant le mieux possible les fibres endommagées. On cherche également à maintenir cet effet pendant une durée prolongée en évitant ainsi un renouvellement trop fréquent des traitements ayant pour inconvénient d'alourdir la chevelure.

La demanderesse a constaté qu'en associant une silicone, un latex et un agent de mise en suspension du latex et de la silicone et/ou un épaississant, il était possible de réparer la fibre capillaire en lui communiquant au même moment de la douceur, une grande facilité de démêlage, un toucher lisse et gainé.

Elle a également constaté que l'association de la silicone, du latex et de l'agent de mise en suspension du latex et de la silicone et/ou de l'épaississant. conduisait à une rémanence des effets par rapport à des compositions contenant uniquement une silicone et à une amélioration de la facilité de démêlage, qui persiste même après un ou plusieurs shampooings, comparativement aux compositions contenant uniquement la silicone ou le latex.

La demande EP-A-0 214 626 décrit une composition capillaire comprenant de 0,01 à 10 % en poids de particules polymériques dont, entre autres, des latex, ainsi qu'éventuellement des huiles de silicone. Ce document ne décrit cependant pas de moyens permettant de maintenir le latex et la silicone en suspension de façon essentiellement uniforme dans la composition.

La présence des agents épaississants particuliers définis ci-après et/ou d'agents de suspension permet d'obtenir une répartition régulière des silicones et du latex, sous forme de suspension homogène, dans la composition de traitement. Ils permettent également une application aisée de la composition sur les matières kératiniques.

Les effets décrits ci-dessus se sont avérés rémanents même après plusieurs shampooings.

L'invention a donc pour objet une composition de traitement des matières kératiniques comprenant dans un milieu approprié pour l'application sur les matières, au moins une silicone, au moins un latex et au moins un agent de mise en suspension de la silicone et du latex et/ou au moins un agent épaississant choisi parmi les gommes de guar, la gomme arabique, les scléroglucanes, les acides polyacryliques réticulés ou non, les dispersions aqueuses des copolymères d'acrylate d'ammonium/acrylamide réticulés et l'émulsion du copolymère d'acrylamide/acrylamide de 2-méthylpropane sulfonique neutralisé.

L'invention a également pour objet un procédé de traitement des matières kératiniques, en particulier des cheveux ou des cils, mettant en oeuvre une composition telle que définie ci-après.

D'autres objets de l'invention apparaîtront à la lecture de la description et des exemples qui suivent.

La composition de traitement des matières kératiniques conforme à l'invention est essentiellement caractérisée par le fait qu'elle contient dans un milieu aqueux, au moins une silicone, au moins un latex insoluble dans le milieu aqueux et un agent de mise en suspension de la silicone et du latex et/ou au moins un agent épaississant choisi parmi les gommes de guar, la gomme arabique, les scléroglucanes, les acides polyacryliques réticulés ou non, les dispersions aqueuses des copolymères d'acrylate d'ammonium/acrylamide réticulés et l'émulsion du copolymère d'acrylamide/acrylamide de 2-méthylpropane sulfonique neutralisé.

Les silicones, utilisées conformément à la présente invention, sont des polyorganosiloxanes insolubles ou solubles dans les milieux aqueux, pouvant se présenter sous forme d'huiles, de cires, de gommes ou de résines.

Les organopolysiloxanes sont définis plus en détail dans l'ouvrage de Walter NOLL "Chemistry and Technology of Silicones" (1968) Academic-Press.

Les polysiloxanes utilisés, conformément à l'invention, sont choisis parmi les silicones volatiles possédant un point d'ébullition compris entre 60 °C et 260 °C, ou bien les silicones non volatiles choisies en particulier parmi les polyalkylsiloxanes, les polyaryl siloxanes, les polyalkylarylsiloxanes, les gommes et résines de silicones, les polysiloxanes organomodifiés ainsi que leurs mélanges.

Les silicones volatiles peuvent être choisies parmi :

(i) les silicones cycliques comportant de 3 à 7 atomes de silicium et de préférence 4 à 5. Il s'agit, par exemple, de l'octaméthylcyclo tétrasiloxane vendu sous le non, de VOLATILE SILICONE 7207 par UNION CARBIDE ou SILBIONE 70045 V 2 par RHONE POULENC, le décaméthylcyclopentasiloxane vendu sous le nom de VOLATILE SILICONE 7158 par UNION CARBIDE, SILBIONE 70045 V 5 par RHONE POULENC, ainsi que leurs mélanges.

On peut citer également les cyclocopolymères du type diméthylsiloxane/méthylalkylsiloxane, tel que la SILICONE VOLATILE FZ 3109 vendue par la Société UNION CARBIDE, qui est un cyclopolymère diméthylsiloxane/méthyloctylsiloxane.

On peut utiliser par ailleurs les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy)bis-néopentane;

(ii) les silicones volatiles linéaires ayant 2 à 9 atomes de silicium et possédant une viscosité inférieure ou égale à $5.10^{-6}$ $m^2/s$ à 25 °C. Il s'agit, par exemple, de l'hexaméthyldisiloxane vendu sous la dénomination SILBIONE 70 041 V 0,65 par la Société RHONE POULENC, du décaméthyltétrasiloxane vendu sous la dénomination SH 200 par la Société TORAY SILICONE ou par les polyméthyl phénylsiloxanes volatils tels que le produit SILICONOL AS vendu par la Société WACKER. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Les silicones non volatiles sont choisies notamment parmi les polyalkylsiloxanes. On peut citer principalement les polydiméthyl siloxanes linéaires à groupements terminaux triméthylsilyle de viscosité $5.10^{-6}$ à 2,5 $m^2/s$ à 25 °C et de préférence $10^{-5}$ à 1 $m^2/s$, comme par exemple et à titre non limitatif :

. les huiles SILBIONE des séries 47 et 70 047 commercialisées par RHONE POULENC, telles que l'huile 47 V 500.000,
. les huiles de la série 200 de la Société DOW CORNING,
. les huiles VISCASIL de la GENERAL ELECTRIC et certaines huiles des séries SF de la GENERAL ELECTRIC (SF 96, SF 18).

On cite également les polydiméthylsiloxanes linéaires à groupements terminaux diméthylsilanol, tels que les huiles de la série 48 de RHONE POULENC.

Les polydiméthylsiloxanes de viscosité supérieure à $10^{-1}$ $m^2/s$ sont préférés.

Dans cette classe de polyalkylsiloxanes, on peut également mentionner les cires de polyalkylsiloxanes vendues par la Société GOLDSCHMIDT sous les dénominations ABIL WAX 9800 et ABIL WAX 9801, qui sont des polyalkyl($C_1$-$C_{20}$)-siloxanes.

Parmi les polyalkylarylsiloxanes, on peut citer les polydiméthyl méthylphénylsiloxanes, les polyméthylphénylsiloxanes, les poly diméthyldiphénylsiloxanes linéaires et/ou ramifiés, de viscosité $10^{-5}$ à $5.10^{-2}$ $m^2/s$ à 25 °C, tels que, par exemple :

. les huiles des séries RHODORSIL 70 633 et 763 de RHONE POULENC,
. les huiles SILBIONE de la série 70 641 de RHONE POULENC,
. l'huile DC 556 COSMETIC GRAD FLUID de DOW CORNING,
. les silicones de la série PK de BAYER, telles que la PK20,
. les silicones des séries PN, PH de BAYER, comme les PN 1000 et PH 1000,
. certaines huiles des séries SF de GENERAL ELECTRIC, telles que les SF 1250, SF 1265, SF 1154, SF 1023.

Les gommes de silicones, conformes à la présente invention, sont des polydiorganosiloxanes de fortes masses moléculaires, comprises entre 200.000 et 1.000.000, utilisées seules ou en mélange dans un solvant choisi parmi les silicones volatiles telles que définies ci-dessus, les huiles polydiméthylsiloxanes (PDMS), les huiles poly phénylméthylsiloxanes (PPMS), les isoparaffines, le chlorure de méthylène, le pentane, le dodécane, le tridécane, le tétradécane ou leurs mélanges.

On cite, par exemple, les gommes suivantes :

- poly[(diméthylsiloxane)/(méthylvinylsiloxane)],
- poly[(diméthylsiloxane)/(diphénylsiloxane)].
- poly[(diméthylsiloxane)/(phénylméthylsiloxane)],
- poly[(diméthylsiloxane)/(diphénylsiloxane)/méthylvinylsiloxane)].

On peut citer, par exemple, à titre non limitatif, les mélanges suivants :

. les mélanges formés à partir d'un polydiméthylsiloxane hydroxylé en bout de chaîne (DIMETHICONOL selon la nomenclature CTFA) et d'un polydiméthylsiloxane cyclique (CYCLOMETHICONE selon la nomenclature CTFA), tel que le produit Q2 1401 vendu par la Société DOW CORNING,

. les mélanges formés à partir d'une gomme polydiméthylsiloxane avec une silicone cyclique, tel que le produit SF 1214 Silicone Fluid de la Société GENERAL ELECTRIC (qui est une gomme SE 30, correspondant à une diméthicone, ayant un poids moléculaire de 500.000 solubilisée dans la silicone SF 1202 Silicone Fluid (correspondant au décaméthylcyclopentasiloxane)),

. les mélanges de deux PDMS de viscosités différentes, notamment d'une gomme PDMS et d'une huile PDMS, tels que les produits SF 1236 et CF 1241 de la Société GENERAL ELECTRIC. Le produit SF 1236 est le mélange d'une gomme SE 30 définie ci-dessus d'une viscosité de 20 $m^2/s$ et d'une huile SF 96 d'une viscosité de $5.10^{-6}$ $m^2/s$ (15% de gomme SE 30 et 85% d'huile SF 96).

Le produit CF 1241 est le mélange d'une gomme SE 30 (33%) et d'une PDMS (67%) de viscosité $10^{-3}$ $m^2/s$.

Les résines d'organopolysiloxanes utilisables conformément à l'invention, sont des systèmes siloxaniques réticulés renfermant les unités $R'_2SiO_{2/2}$, $R'SiO_{3/2}$ et $SiO_{4/2}$, dans lesquelles R' représente un groupement hydrocarboné possédant 1 à 6 atomes de carbone ou un groupement phényle. Parmi ces produits, ceux particulièrement préférés sont ceux dans lesquels R' désigne un radical alkyle inférieur ou un radical phényle.

Parmi ces résines, on peut citer le produit vendu sous la dénomination DOW CORNING 593 ou ceux vendus sous les dénominations SILICONE FLUID SS 4230 et SS 4267 par la Société GENERAL ELECTRIC et qui sont des "diméthyl/triméthylpoly siloxane".

Les silicones organomodifiées sont des silicones définies ci-dessus et comportant dans leur structure un ou plusieurs groupements organo-fonctionnels directement fixés sur la chaîne siloxanique ou fixés par l'intermédiaire d'un radical hydrocarboné.

Parmi ces silicones organomodifiées, on peut citer, par exemple, les silicones comportant :

**1-** des groupements polyéthylèneoxy et/ou polypropylèneoxy, comportant éventuellement des groupes alkyles, tels que :

. le produit dénommé diméthicone copolyol vendu par la Société DOW CORNING sous les dénominations DC 1248, et l'alkyl($C_{12}$) méthicone copolyol vendue par la Société DOW CORNING sous la dénomination Q2 5200,

. les huiles SILWET L 722, L 7500, L 77, L 711 de la Société UNION CARBIDE.

. le mélange de diméthicone copolyol et de cyclométhicone tels que le produit vendu sous la dénomination Q2-3225C par la Société DOW CORNING,

**2-** des groupements aminés substitués ou non comme les produits vendus sous la dénomination GP4 Silicone Fluid et GP 7100 par la Société GENESEE ou les produits vendus sous les dénominations Q2 8220, X2 8200 et DC 929 ou Q2 7224 par la Société DOW CORNING. Les groupements aminés substitués sont en particulier des groupements aminoalkyle($C_1$-$C_4$),

**3-** des groupements thiols comme dans les GP 72 A et GP 71 de GENESEE ou dans le produit SLM 50253/5 de la Société WACKER,

**4-** des groupements carboxylates comme dans les produits décrits dans le brevet EP-A-186.507 de la Société CHISSO CORPORATION,

**5-** des groupements alkoxylés comme les produits vendus sous la dénomination SILICONE COPOLYMER F-755 par SWS SILICONES, et ABIL WAX 2428, 2434 et 2440 par la Société GOLDSCHMIDT.

**6-** des groupements hydroxylés, comme les polyorganosiloxanes à fonction hydroxyalkyle, décrits dans la demande de brevet français n° FR-85.16.334, répondant à la formule suivante (I) :

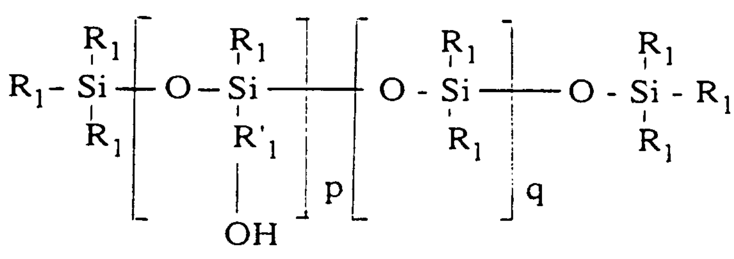

(I)

dans laquelle :

les radicaux $R_1$, identiques ou différents, sont choisis parmi les radicaux méthyle et phényle, au moins 60% en mole des radicaux $R_1$ désignant méthyle;

le radical $R'_1$ est un chaînon alkylène divalent hydrocarboné en $C_2$-$C_{18}$;

p est compris entre 1 et 30 inclus;

q est compris entre 1 et 150 inclus.

On cite, par exemple, le produit 71615 V 300 vendu par la Société RHONE POULENC.

**7-** des groupements acyloxyalkyle, comme par exemple les polyorgano siloxanes décrits dans la demande de brevet FR-A-2.641.185, répondant à la formule (II) :

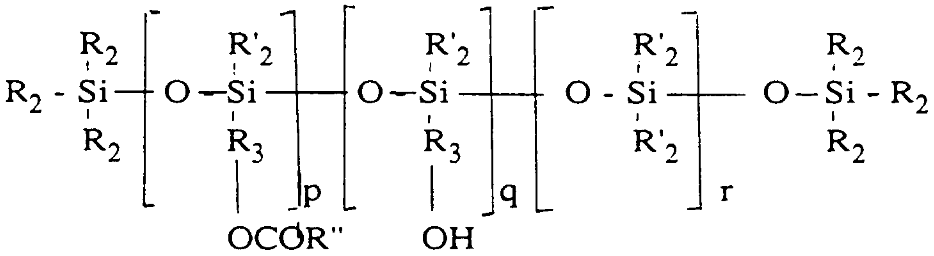 (II)

dans laquelle :

$R_2$ désigne un groupement méthyle, phényle, -OCOR", hydroxyle, un seul des radicaux $R_2$ par atome de silicium peut être OH;

$R'_2$ désigne méthyle, phényle, au moins 60% en mole de l'ensemble des radicaux $R_2$ et $R'_2$ désignant méthyle;

R" désigne un alkyle ou alkényle en $C_8$-$C_{20}$;

$R_3$ désigne un radical alkylène hydrocarboné divalent, linéaire ou ramifié, en $C_2$-$C_{18}$;

r est compris entre 1 et 120 inclus;

p est compris entre 1 et 30;

q est égal à 0 ou est inférieur à 0,5p, p+q étant compris entre 1 et 30; les polyorganosiloxanes de formule (II) pouvant contenir des groupements

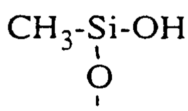

dans des proportions ne dépassant pas 15% de la somme p+q+r.

**8-** des groupements anioniques de type carboxylique tels que les groupements alkylcarboxyliques comme ceux présents dans le produit X-22-3701E de la Société SHIN-ETSU ou dans le produit Silicone Fluid FZ 3703 de la Société UNION CARBIDE; 2-hydroxyalkylsulfo nate; 2-hydroxyalkylthiosulfate tels que les produits vendus par la Société GOLDSCHMIDT sous les dénominations "ABIL S201" et "ABIL S255".

Les polyorganosiloxanes plus particulièrement préférés, conformément à l'invention, sont :

. les huiles de silicone de forte viscosité comprise entre 0,2 et 2,5 $m^2/s$ à 25 °C, telles que les huiles 47 V 500.000 de la Société RHONE POULENC,

. les mélanges d'organopolysiloxanes et de silicones cycliques, tels que le produit Q2 1401 vendu par la Société DOW CORNING,

. les mélanges de deux PDMS de viscosités différentes, tels que le produit vendu par la Société GENERAL ELECTRIC sous la dénomination CF 1241.

Les latex sont des suspensions colloïdales de particules de polymère dans une phase liquide aqueuse. Ces latex sont obtenus généralement par polymérisation ou copolymérisation en suspension ou en émulsion de monomères selon les procédés bien connus dans l'état de la technique.

Les latex utilisés conformément à l'invention résultent, en particulier, de la polymérisation ou de la copolymérisation de monomères tels que le styrène, le butadiène, l'acrylonitrile, le chloroprène, l'acétate de vinyle, les uréthanes, l'isoprène. l'isobutylène et les acides acrylique ou méthacrylique, maléïque, crotonique, itaconique ou leurs esters ou amides.

Ces latex sont insolubles dans le milieu aqueux et doivent posséder en plus les propriétés filmogènes et restructurantes.

Selon l'invention, on appelle un latex filmogène un latex dont 100 ml d'une solution à 8 g/100 ml déposés dans une matrice rigoureusement horizontale de 65 $cm^2$ de surface, laisse apparaître à l'oeil nu après 15 heures de séchage à la température ambiante, une fine pellicule homogène et uniforme sur toute la surface de la matrice.

On appelle un latex restructurant un latex qui, lorsqu'il est appliqué sur la surface de la fibre capillaire, restitue à la fibre sa conformation d'origine. Le terme restructurant n'est pas utilisé pour désigner une modification de la kératine elle-même.

Les latex plus particulièrement utilisés conformément à l'invention sont :

- des homopolymères et des copolymères d'acétate de vinyle, tels que :
  le polyacétate de vinyle comme les produits vendus sous les dénominations "Emulsion RHODOPAS A012P", "Emulsion RHODOPAS A013P" par la Société RHONE POULENC;
  les copolymères d'acétate de vinyle et d'éthylène, tels que les produits vendus sous les dénominations "APPRETAN MB", "APPRETAN EM", "APPRETAN TV" par la Société HOECHST;
- des homopolymères ou des copolymères dérivés d'acide acrylique, tels que les produits vendus sous les dénominations "PRIMAL AC-33", "PRIMAL K-3", "PRIMAL TR-93", "PRIMAL HA-8", "PRIMAL E-358", commercialisés par la Société ROHM & HAAS ou RHODOPAS SD215 commercialisé par la Société RHONE POULENC;
- des polyuréthanes, tels que ceux vendus sous la dénomination "WITCOBOND 160", par la Société WITCO;
- des copolymères butadiène/styrène, carboxylés ou non, tels que les produits vendus sous les dénominations "Emulsion RHODOPAS SB02, "Emulsion RHODOPAS ST246", "Emulsion RHODOPAS SB 153", "Emulsion RHODOPAS GB012" par la Société RHONE POULENC;
- des copolymères butadiène/acrylonitrile, carboxylés ou non, tels que les produits vendus sous les dénominations "HYCAR 1562" par la Société GOODRICH et "CHEMIGUM L6271" par la Société GOODYEAR;
- des copolymères styrène/esters acryliques tels que le produit vendu sous la dénomination "APPRETAN V3749" par la Société HOECHST.

Parmi les agents épaississants, le copolymère d'acrylate d'ammonium/acrylamide réticulé, en dispersion aqueuse, est réticulé par un agent de réticulation à polyinsaturation oléfinique, et dispersé dans une émulsion eau-dans-huile comprenant la paraffine et un mélange de stéarate de sorbitan et de dérivé éthoxylé hydrophile. Il s'agit plus particulièrement de l'émulsion commercialisée sous la dénomination de PAS 5161 par la Société HOECHST, comprenant le copolymère acrylate d'ammonium/acrylamide (95/5 en poids), le milieu étant constitué par 30% en poids dudit polymère, 25% en poids de paraffine, 4% de mélange de sorbitan et de dérivé éthoxylé hydrophile et 41% d'eau.

Les agents de mise en suspension sont choisis parmi les composés de formule :

a) $R_4X$ (III)

dans laquelle $R_4$ est un radical aliphatique à longue chaîne carbonée, éventuellement interrompue par des atomes d'oxygène, et X est un reste acide carboxylique, sulfurique ou phosphorique ou un radical dérivé d'un acide carboxylique ou d'un amide; ces composés de formule (III) sont choisis parmi ceux dans lesquels :

(i) $R_4$ est un radical alkyle ou alcényle en $C_{11}$-$C_{21}$

X est :

. un groupement COOA où A est un radical mono ou polyhydroxy alkyle dérivé d'un polyol en $C_2$-$C_3$ ou un radical $CH_2CH_2SO_3M$,

. un groupement $CO(OCH_2CH_2)_n$-OH où n a une valeur comprise entre 2 et 150.

. un groupement

$$COOCH_2\text{-}\underset{\displaystyle CH_3}{\underset{|}{C}H}\text{-}(OCH_2CH_2)_nOH$$

où n a une valeur comprise entre 2 et 150, les fonctions OH libres des groupements définis ci-dessus pouvant être estérifiées par un acide $R_4COOH$ où $R_4$ est un alkyle ou un alcényle en $C_{11}$-$C_{21}$,

. un groupement $CONR_5R_6$ où $R_5$ et $R_6$ représentent hydrogène ou hydroxyalkyle en $C_1$-$C_4$, l'un au moins représentant hydroxyalkyle en $C_1$-$C_4$,

. un groupement $OSO_3M$ ou 1/3 $PO_4^{3\ominus}M_3$ où M représente un métal alcalin, ammonium ou un reste d'alcanolamine en $C_1$-$C_4$;

(ii) $R_4$ désigne un radical $R_7(OC_2H_4)_\ell$ $OCH_2$ et X désigne un groupement COOM où M a la signification indiquée ci-dessus, $R_7$ désignant un radical alkyle en $C_{12}$-$C_{14}$ et $\ell$ un nombre entier ou décimal compris entre 2,5 et 10, ou bien $R_7$ désigne oléyle et $\ell$ varie de 2 à 9 ou encore $R_7$ désigne

alkyl($C_8$-$C_9$)phényle et ℓ varie de 4 à 8, ou les dérivés dans lesquels $R_4$ désigne un groupement alkyl($C_{12}$-$C_{16}$) éther et X un groupement $CONR_5R_6$, dans lequel $R_5$ et $R_6$ ont la même signification que celle indiquée ci-dessus;

b) des oxydes de diméthylalkyl($C_{16}$-$C_{22}$)amines.

Un autre agent de suspension utilisable selon l'invention est un alcool ayant 27 à 44 atomes de carbone et comportant un ou deux groupements éther et/ou thioéther ou sulfoxyde, répondant à la formule (IV) :

$$R_8 - X -[C_2H_3(OH)]-CH_2- Y - R_9 \qquad (IV)$$

dans laquelle :

$R_8$ et $R_9$, identiques ou différents, désignent des groupements alkyle linéaires en $C_{12}$ à $C_{20}$;

X désigne un atome d'oxygène, un atome de soufre ou un groupement sulfoxyde;

Y désigne un atome d'oxygène, un atome de soufre, un groupement sulfoxyde ou un groupement méthylène;

dans le cas où Y désigne un groupement méthylène, la somme des nombres d'atomes de carbone de $R_8$ à $R_9$ varie de 24 à 40, et de préférence de 26 à 36 inclus, et lorsque Y ne désigne pas un groupement méthylène, la somme des atomes de carbone de $R_8$ et $R_9$ varie de 24 à 40 inclus, et de préférence de 28 à 36 inclus; lorsque X ou Y désigne sulfoxyde, Y ou X ne désigne pas soufre.

Les compositions plus particulièrement préférées, conformément à l'invention, contiennent une silicone insoluble tel qu'un mélange formé à partir d'un polydiméthylsiloxane hydroxylé en bout de chaîne dénommé encore DIMETHICONOL selon la nomenclature CTFA et d'un polydiméthylsiloxane cyclique dénommé encore CYCLOMETHICONE selon la nomenclature CTFA, un latex qui est un homopolymère ou un copolymère dérivé de l'acide acrylique, la composition contenant comme agent épaississant, de façon préférentielle, une dispersion aqueuse de copolymère d'acrylate d'ammonium et d'acrylamide réticulé défini ci-dessus.

La ou les silicones sont utilisées dans les compositions conformes à l'invention dans des proportions comprises entre 0,1 et 50% en poids par rapport au poids total de la composition, et de préférence entre 0,2 et 30% en poids; le latex est présent de préférence dans des proportions comprises entre 0,1 et 10% en poids et en particulier entre 0,5 et 5% en poids; l'agent épaississant ou de mise en suspension est utilisé dans des proportions suffisantes pour épaissir ou mettre en suspension la ou les silicones et le ou les latex, ces proportions étant comprises de préférence entre 0,1 et 20% en poids et en particulier entre 0,3 et 10% en poids par rapport au poids total de la composition.

Les compositions conformes a l'invention peuvent contenir différents adjuvants suivant l'application envisagée.

Lorsque ces compositions sont utilisées sous forme de shampooing, elles contiennent en plus des agents tensio-actifs détergents de nature anionique, non-ionique, amphotère ou zwittérionique ou leurs mélanges dans des proportions comprises entre 5 et 50% en poids et de préférence entre 8 et 35% en poids par rapport au poids total de la composition.

Les compositions de traitement peuvent également contenir des polymères autres que des latex et notamment des polymères cosmétiquement acceptables, des protéines, ces polymères étant choisis et utilisés dans des proportions de façon à ne pas altérer la stabilité des compositions.

Les compositions conformes à l'invention peuvent également contenir d'autres adjuvants habituellement utilisés en cosmétique, tels que des parfums, des conservateurs, des séquestrants, des stabilisateurs de mousse, des agents propulseurs, des colorants, des agents acidifiants ou alcalinisants, suivant l'application envisagée.

Lorsque les compositions sont utilisées dans un but de traitement thérapeutique tel que de la peau ou des cheveux, elles contiennent en plus, des substances actives pour le traitement des affections dermatologiques ou du cheveu.

Les compositions selon l'invention sont utilisables également comme lotion traitante à rincer ou non, à appliquer avant ou après un shampooing, avant ou après une permanente, avant ou après une coloration ou décoloration ou entre les deux étapes d'une permanente ou d'un défrisage.

Les compositions selon l'invention sont utilisables également dans le maquillage des cils et notamment comme mascaras. Dans ce cas, elles peuvent contenir en outre des pigments, des huiles ou cires animales, végétales ou minérales, des alcools gras, des esters d'acides gras, des acides gras. Ces compositions permettent un maquillage aisé, uniforme et ayant de la tenue dans le temps.

Le procédé de traitement conforme à l'invention consiste à appliquer sur les matières kératiniques destinées à être traitées, la composition telle que définie ci-dessus dans des proportions suffisantes pour réparer les fibres capillaires et pour leur conférer les propriétés de douceur, de démêlage recherchées.

Lorsqu'elles sont utilisées comme shampooing, elles sont appliquées sur des cheveux mouillés et après un temps de pose de quelques minutes, les cheveux sont rincés et séchés.

Les exemples qui suivent sont destinés à illustrer l'invention sans pour autant présenter un caractère limitatif.

EXEMPLE 1

On prépare un après-shampooing non rincé de composition suivante :

| | |
|---|---|
| - Mélange de diméthiconol (13%) d'octaméthylcyclotétrasiloxane et dodécaméthylcyclopentasiloxane (87%) vendu sous la dénomination Q2 1401 par la Société DOW CORNING | 20,0 g |
| - Dispersion aqueuse de polyacétate de vinyle vendue à 54% de MA sous la dénomination Emulsion RHODOPAS A 012P par la Société RHONE POULENC | 1,0 g MA |
| - Emulsion de copolymère acrylate d'ammonium/acrylamide réticulé vendue sous la dénomination PAS 5161 par la Société HOECHST | 0,3 g MA |
| - Conservateur, parfum qs | |
| - Acide chlorhydrique qs pH = 6 | |
| - Eau | qsp 100,0 g |

EXEMPLE 2

On prépare un après-shampooing à rincer de composition suivante :

| | |
|---|---|
| - Mélange de deux PDMS de viscosités différentes vendu sous la dénomination CF 1241 par la Société GENERAL ELECTRIC | 3,0 g |
| - Emulsion aqueuse de polyuréthane vendu à 35% de MA sous la dénomination WITCOBOND 160 par la Société WITCO | 1,8 g MA |
| - Emulsion H/E de copolymère acrylamide/acrylamide de 2-méthylpropane sulfonate de sodium, vendue à 35-45% de MA par la Société SEPPIC sous la dénomination SEPIGEL 305 | 1,5 g MA |
| - Conservateur, parfum qs | |
| - Acide chlorhydrique qs pH = 8 | |
| - Eau | qsp 100,0 g |

EXEMPLE 3

On prépare un après-shampooing à rincer de composition suivante :

| | |
|---|---|
| - Polydiméthylsiloxane vendu sous la dénomination HUILE 47 V 500.000 par la Société RHONE POULENC | 2,0 g |
| - Dispersion aqueuse de copolymère butadiène/acrylonitrile vendue sous la dénomination HYCAR 1562 par la Société GOODRICH à 41% de MA | 5,0 g MA |
| - Alginate de sodium vendu sous la dénomination ALGINATE MP/8 par la Société Française des Colloïdes | 4,0 g |
| - Conservateur, parfum qs | |
| - Acide chlorhydrique qs pH = 7 | |
| - Eau | qsp 100,0 g |

EXEMPLE 4

On prépare un après-shampooing à rincer de composition suivante :

| | |
|---|---|
| - Polydiméthylsiloxane vendu sous la dénomination HUILE 47V 500.000 par la Société RHONE POULENC | 20,0 g |
| - Emulsion acrylique aqueuse vendue à 46% de MA par la Société ROHM & HAAS sous la dénomination PRIMAL HA 8 | 0,5 g MA |
| - Gomme de guar | 2,0 g |
| - Conservateur, parfum qs | |
| - Hydroxyde de sodium qs pH = 6 | |
| - Eau | qsp 100,0 g |

EXEMPLE 5

On prépare un après-shampooing non rincé, de composition suivante :

| | |
|---|---|
| - Polydiméthylsiloxane vendu sous la dénomination HUILE 47V 500.000 par la Société RHONE POULENC | 5,0 g |
| - Dispersion aqueuse de copolymère styrène/esters acryliques autoréticulable, vendue sous la dénomination APPRETAN V 3749 par la Société HOECHST | 10,0 g |
| - Acide polyacrylique réticulé, vendu sous la dénomination CARBOPOL 940 par la Société GOODRICH | 0,9 g |
| - Conservateur qs | |
| - Triéthanolamine qs pH = 4,2 | |
| - Eau | qsp 100,0 g |

EXEMPLE 6

On prépare un shampooing de composition suivante :

| | |
|---|---|
| - Lauryléthersulfate de sodium oxyéthyléné à 2 moles d'oxyde d'éthylène à 28% de MA | 16,8 g MA |
| - Cocoylbétaïne à 32% de MA | 2,6 g MA |
| - Composé de formule (IV) dans laquelle :<br>$R_8$ désigne $C_{16}H_{33}$<br>$R_9$ désigne $C_{14}H_{29}$<br>X désigne O<br>Y désigne $CH_2$ | 2,5 g |
| préparé par réaction de 3 moles d'alcool sur 1 mole d'époxyde, utilisé brut | |
| - Mélange d'isopropanolamides d'acide de coprah, vendu sous la dénomination EMPILAN CIS par la Société MARCHON | 1,0 g |
| - Cire de Lanette | 0,75 g |
| - Polydiméthylsiloxane vendu sous la dénomination HUILE 47 V 500.000 par la Société RHONE POULENC | 3,0 g |
| - Emulsion acrylique aqueuse, vendue sous la dénomination PRIMAL K-3 par la Société ROHM & HAAS | 3,0 g |
| - Conservateur, parfum qs | |
| - pH spontané = 5,1 | |
| - Eau | qsp 100,0 g |

EXEMPLE 7

On prépare un après-shampooing non rincé de composition suivante :

| | |
|---|---|
| - Emulsion H/E de copolymère acrylamide/acrylamide de 2-méthylpropane sulfonate de sodium, vendue à 35-45% de MA par la Société SEPPIC sous la dénomination SEPIGEL 305 | 0,8 g MA |
| - Latex de copolymère acétate de vinyle/esters acryliques vendu à 54% de MA sous la dénomination RHODOPAS AD 310 par la Société RHONE POULENC | 1,0 g MA |
| - Mélange de diméthiconol (13%), d'octaméthylcyclotétrasiloxane et de décaméthylcyclopentasiloxane (87%), vendu sous la dénomination Q2-1401 par la Société DOW CORNING | 20,0 g |
| - Conservateur qs | |
| - pH spontané = 4,9 | |
| - Eau | qsp 100,0 g |

EXEMPLE 8

**MASCARA**

| | |
|---|---|
| - Stéarate de triéthanolamine | 13,0 g |
| - Cire d'abeille | 12,0 g |
| - Cire de Candelilla | 3,0 g |
| - Emulsion de copolymère acrylate d'ammonium/acrylamide réticulé, vendu sous la dénomination PAS 5161 par la Sociét HOESCHT | 0,15 g |
| - Gomme arabique | 1,0 g |
| - Polyvinylpyrrolidone | 1,0 g |
| - Copolymère ester acrylique/styrène en dispersion anionique à 50%, vendu sous la dénomination RHODOPAS SD 215 par la Société RHONE POULENC | 4,0 g |
| - Oxydes de fer noir | 7,0 g |
| - Mélange de diméthiconol et de cyclométhicone vendu sous la dénomination SILICONE Q2 1401 par DOW CORNING | 5,0 g |
| - Conservateurs | 0,4 g |
| - Eau | qsp 100,0 g |

On chauffe la phase grasse à 85 °C et on ajoute les oxydes de fer. La phase aqueuse contenant les différents composés hydrosolubles est chauffée à 85 °C. Sous vive agitation, on ajoute la phase aqueuse à la phase grasse en mélangeant à la turbine. A 60 °C, on ajoute le mélange de silicones puis on laisse refroidir jusqu'à température ambiante.

Ce mascara est facile à appliquer sur les cils et confère un maquillage uniforme et durable.

**Revendications**

**1.** Composition de traitement des matières kératiniques, caractérisée par le fait qu'elle contient dans un milieu aqueux, au moins une silicone, au moins un latex insoluble dans le milieu aqueux et au moins un agent de mise en suspension de la silicone et du latex et/ou au moins un agent épaississant choisi parmi les gommes de guar, la gomme arabique, les scléroglucanes, les acides polyacryliques réticulés ou non, les dispersions aqueuses des copolymères d'acrylate d'ammonium/acrylamide réticulés et l'émulsion du copolymère d'acrylamide/acrylamide de 2-méthylpropane sulfonique neutralisé .

**2.** Composition selon la revendication 1, caractérisée par le fait que la silicone est choisie parmi les polyorganosiloxanes insolubles ou solubles dans le milieu aqueux et se présente sous forme d'huile, de cire, de gomme ou de résine.

**3.** Composition selon la revendication 2, caractérisée par le fait que les polysiloxanes sont choisis parmi les silicones volatiles ayant un point d'ebullition compris entre 60 °C et 260 °C.

**4.** Composition selon la revendication 3, caractérisée par le fait que les silicones volatiles sont choisies parmi :

- les silicones cycliques comportant de 3 à 7 atomes de silicium;
- les cyclopolymères du type diméthylsiloxane/méthylalkylsiloxane;
- les mélanges de silicones cycliques avec des composés organiques dérivés du silicium;
- les silicones volatiles linéaires ayant 2 à 9 atomes de silicium et de viscosité inférieure ou égale à $5.10^{-6}$ $m^2/s$ à 25 °C.

**5.** Composition selon l'une quelconque des revendications 1 ou 2, caractérisée par le fait que les silicones non volatiles sont choisies parmi :

(a) les polyalkylsiloxanes choisis parmi :

- les polydiméthylsiloxanes linéaires à groupements terminaux triméthylsilyle de viscosité comprise entre $5.10^{-6}$ et 2,5 $m^2/s$ à 25 °C;
- les polydiméthylsiloxanes linéaires à groupements terminaux diméthylsilanol;
- les polyalkyl($C_1$-$C_{20}$)siloxanes;

(b) les polyalkylarylsiloxanes choisis parmi :

- les polydiméthylméthylphénylsiloxanes, les polyméthylphénylsiloxanes, les polydiméthyldiphénylsiloxanes linéaires et/ou ramifiés, de viscosité comprise entre $1.10^{-5}$ et $5.10^{-2}$ $m^2/s$ à 25 °C;

(c) les gommes de silicone, choisies parmi les polydiorganosiloxanes ayant des masses moléculaires comprises entre 200 000 et 1 000 000 utilisées seules ou sous forme de mélange dans un solvant;

(d) les résines de silicone constituées d'unités :

$R'_2\ Si\ O_{2/2}$, $R'\ Si\ O_{3/2}$, $Si\ O_{4/2}$

dans lesquelles R' représente un groupement hydrocarboné ayant de 1 à 6 atomes de carbone ou un groupement phényle;

(e) les silicones organomodifiées, choisies parmi les silicones comportant dans leur structure un ou plusieurs groupements organofonctionnels directement fixés sur la chaîne siloxanique ou fixés par l'intermédiaire d'un radical hydrocarboné.

**6.** Composition selon la revendication 5, caractérisée par le fait que les gommes de silicone utilisées seules ou sous forme de mélange sont choisies parmi les structures suivantes :

- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/méthylvinylsiloxane)]

et les mélanges suivants :

- des mélanges formés à partir d'un polydiméthylsiloxane hydroxylé en bout de chaîne et d'un polydiméthylsiloxane cyclique;
- les mélanges formés à partir d'une gomme polydiméthylsiloxane et d'une silicone cyclique; et
- des mélanges de polydiméthylsiloxanes de viscosités différentes.

**7.** Composition selon la revendication 5, caractérisée par le fait que les silicones organomodifiées sont choisies parmi les polyorganosiloxanes comportant :

a) des groupements polyéthylèneoxy et/ou polypropylèneoxy;
b) des groupements aminés substitués ou non;
c) des groupements thiols;
d) des groupements carboxylates;
e) des groupements alkoxylés;

f) des groupements hydroxyalkyle répondant à la formule suivante :

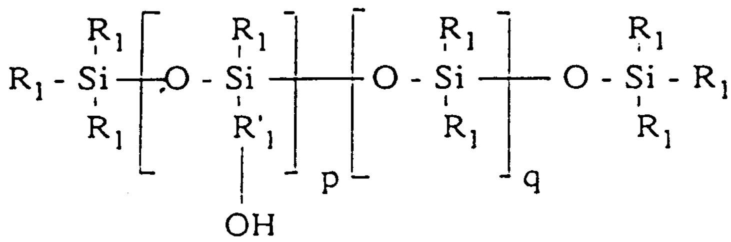

(I)

dans laquelle :

les radicaux $R_1$, identiques ou différents, sont choisis parmi les radicaux méthyle et phényle, au moins 60% en mole des radicaux $R_1$ désignant méthyle;

le radical $R'_1$ est un chaînon alkylène divalent hydrocarboné en $C_2$-$C_{18}$;

p est compris entre 1 et 30 inclus;

q est compris entre 1 et 150 inclus;

g) des groupements acyloxyalkyle répondant à la formule suivante :

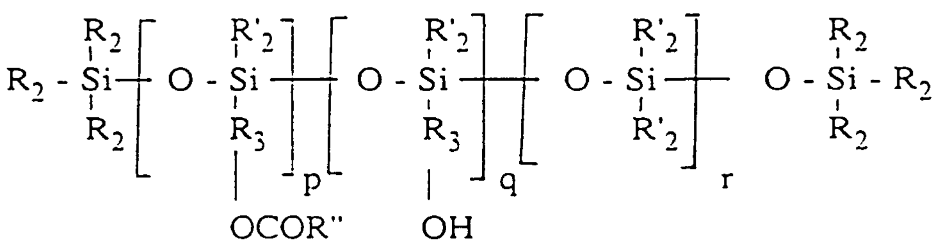

(II)

dans laquelle :

$R_2$ désigne un groupement méthyle, phényle, -OCOR'', hydroxyle, un seul des radicaux $R_2$ par atome de silicium peut être OH;

$R'_2$ désigne méthyle, phényle, au moins 60% en mole de l'ensemble des radicaux $R_2$ et $R'_2$ désignant méthyle;

R'' désigne un alkyle ou alkényle en $C_8$-$C_{20}$;

$R_3$ désigne un radical alkylène hydrocarboné divalent, linéaire ou ramifié, en $C_2$-$C_{18}$;

r est compris entre 1 et 120 inclus;

p est compris entre 1 et 30;

q est égal à 0 ou est inférieur à 0,5p, p + q étant compris entre 1 et 30; les polyorganosiloxanes de formule (II) pouvant contenir des groupements

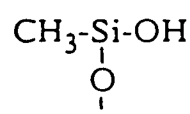

dans des proportions ne dépassant pas 15% de la somme p + q + r;

h) des groupements alkylcarboxyliques,

i) des groupements 2-hydroxyalkylsulfonates,

j) des groupements 2-hydroxyalkylthiosulfates.

**8.** Composition selon l'une quelconque des revendications 1 ou 2, caractérisée par le fait que les polyorganosiloxanes sont choisis parmi les polyalkylsiloxanes linéaires à groupements terminaux triméthyl silyle de viscosité comprise entre 0,2 et 2,5 $m^2/s$ à 25 °C, les mélanges d'un polydiméthylsiloxane hydroxylé en bout de chaîne et d'un polydiméthylsiloxane cyclique, les mélanges de deux polydimethylsiloxanes constitués d'une gomme et d'une huile de viscosités différentes.

**9.** Composition selon l'une quelconque des revendications 1 à 8, caractérisée par le fait que le latex qui est une suspension colloïdale de particules de polymère est obtenu par polymérisation, copolymérisation de monomères choisis parmi le styrène, le butadiène, l'acrylonitrile, le chloroprène, l'acétate de vinyle, les uréthanes, l'isoprène, l'isobutylène, les acides acrylique, méthacrylique, maleïque, crotonique, itaconique ou leurs esters ou leurs amides.

**10.** Composition selon la revendication 9, caractérisée par le fait que le latex insoluble dans le milieu aqueux est choisi parmi les homopolymères et copolymères d'acétate de vinyle; les homopolymères et copolymères d'acide acrylique; les polyuréthanes; les copolymères butadiène/styrène, carboxylés ou non; les copolymères butadiène/acrylonitrile, carboxylés ou non, les copolymères styrène/esters acryliques.

**11.** Composition selon l'une quelconque des revendications 1 à 10, caractérisée par le fait que l'agent de suspension est choisi parmi les composés de formule :

a) $R_4X$ (III)

dans laquelle $R_4$ est un radical aliphatique à longue chaîne carbonée, éventuellement interrompue par des atomes d'oxygène, et X est un reste acide carboxylique, sulfurique ou phosphorique ou un radical dérivé d'un acide carboxylique ou d'un amide; ces composés de formule (III) sont choisis parmi ceux dans lesquels :

(i) $R_4$ est un radical alkyle ou alcényle en $C_{11}$-$C_{21}$

X est :

. un groupement COOA où A est un radical mono ou polyhydroxy alkyle dérivé d'un polyol en $C_2$-$C_3$ ou un radical $CH_2CH_2SO_3M$,

. un groupement $CO(OCH_2CH_2)_n$-OH où n a une valeur comprise entre 2 et 150.

. un groupement

$$COOCH_2\text{-}\underset{CH_3}{CH}\text{-}(OCH_2CH_2)_nOH$$

où n a une valeur comprise entre 2 et 150, les fonctions OH libres des groupements définis ci-dessus pouvant être estérifiées par un acide $R_4COOH$ où $R_4$ est un alkyle ou un alcényle en $C_{11}$-$C_{21}$,

. un groupement $CONR_5R_6$ où $R_5$ et $R_6$ représentent hydrogène ou hydroxyalkyle en $C_1$-$C_4$, l'un au moins représentant hydroxyalkyle en $C_1$-$C_4$,

. un groupement $OSO_3M$ ou 1/3 $PO_4^{3e}M_3$ où M représente un métal alcalin, ammonium ou un reste d'alcanolamine en $C_1$-$C_4$;

(ii) $R_4$ désigne un radical $R_7(OC_2H_4)_\ell$ $OCH_2$ et X désigne un groupement COOM où M a la signification indiquée ci-dessus, $R_3$ désignant un radical alkyle en $C_{12}$-$C_{14}$ et $\ell$ un nombre entier ou décimal compris entre 2,5 et 10, ou bien $R_7$ désigne oléyle et $\ell$ varie de 2 à 9 ou encore $R_7$ désigne alkyl($C_8$-$C_9$)phényle et $\ell$ varie de 4 à 8, ou les dérivés dans lesquels $R_4$ désigne un groupement alkyl($C_{12}$-$C_{16}$) éther et X un groupement $CONR_5R_6$, dans lequel $R_5$ et $R_6$ ont la même signification que celle indiquée ci-dessus;

b) des oxydes de diméthylalkyl($C_{16}$-$C_{22}$)amines.

**12.** Composition selon l'une quelconque des revendications 1 à 11, caractérisée par le fait que l'agent de suspension est un alcool ayant 27 à 44 atomes de carbone et comportant un ou deux groupements éther et/ou thioéther ou sulfoxyde, répondant à la formule (IV) :

$R_8$ - X -[$C_2H_3(OH)$]-$CH_2$- Y - $R_9$ (IV)

dans laquelle :

$R_8$ et $R_9$, identiques ou différents, désignent des groupements alkyle linéaires en $C_{12}$ à $C_{20}$;

X désigne un atome d'oxygène, un atome de soufre ou un groupement sulfoxyde;

Y désigne un atome d'oxygène, un atome de soufre, un groupement sulfoxyde ou un groupement méthylène;

dans le cas où Y désigne un groupement méthylène, la somme des nombres d'atomes de carbone

de $R_8$ à $R_9$ varie de 24 à 40, et de préférence de 26 à 36 inclus, et lorsque Y ne désigne pas un groupement méthylène, la somme des atomes de carbone de $R_8$ et $R_9$ varie de 24 à 40 inclus, et de préférence de 28 à 36 inclus; lorsque X ou Y désigne sulfoxyde, Y ou X ne désigne pas soufre.

**13.** Composition selon l'une quelconque des revendications 1 à 12, caractérisée par le fait que la composition comprend un mélange d'un polydiméthylsiloxane hydroxylé en bout de chaîne et d'un poly diméthylsiloxane cyclique, d'un latex qui est un homopolymère ou copolymère dérivé d'acide acrylique et d'un agent épaississant qui est un copolymère d'acrylate d'ammonium et d'acrylamide réticulé.

**14.** Composition selon l'une quelconque des revendications 1 à 13, caractérisée par le fait que la ou les silicones sont présents dans des proportions comprises entre 0,1 et 50% en poids, de préférence entre 0,2 et 30% en poids par rapport au poids total de la composition.

**15.** Composition selon l'une quelconque des revendications 1 à 14, caractérisée par le fait que le latex est présent dans la composition dans des proportions comprises entre 0,1 et 10% en poids, de préférence entre 0,5 et 5% en poids par rapport au poids total de la composition.

**16.** Composition selon l'une quelconque des revendications 1 à 15, caractérisée par le fait que l'agent épaississant et/ou l'agent de suspension sont présents dans les compositions dans des proportions comprises entre 0,1 et 20% en poids, de préférence entre 0,3 et 10% en poids par rapport au poids total de la composition.

**17.** Composition selon l'une quelconque des revendications 1 à 16. caractérisée par le fait qu'elle se présente sous forme de shampooing contenant en plus au moins un agent tensio-actif détergent choisi parmi les agents tensio-actifs anioniques, non-ioniques, amphotères ou zwittérioniques ou leurs mélanges.

**18.** Procédé de traitement cosmétique des matières kératiniques, caractérisé par le fait que l'on applique sur ces matières au moins une composition telle que définie dans l'une quelconque des revendications 1 à 17.

**19.** Utilisation comme shampooing d'une composition définie dans la revendication 17.

**20.** Utilisation comme mascara d'une composition telle que définie dans l'une quelconque des revendications 1 à 16.

**Claims**

**1.** A composition for treatment of keratinous materials characterised in that it contains in an aqueous medium at least one silicone, at least one latex insoluble in the aqueous medium and at least one suspension agent for the silicone and the latex and/or at least one thickening agent selected from guar gum, gum arabic, scleroglucanes, polyacrylic acids which may be crosslinked or not, aqueous dispersions of crosslinked ammonium acrylate/acrylamide copolymers and an emulsion of neutralised acrylamide/2-methylpropane sulfonic acrylamide copolymer.

**2.** Composition according to claim 1 characterised in that the silicone is selected from polyorganosiloxanes which are soluble or insoluble in the aqueous medium and are in the form of an oil, a wax, a gum or a resin.

**3.** Composition according to claim 2 characterised in that the polysiloxanes are selected from volatiles silicones with a boiling point between 60 °C and 260 °C.

**4.** Composition according to claim 3 characterised in that the volatile silicones are selected from:
- cyclic silicones comprising three to seven silicon atoms;
- dimethylsiloxane/methylalkylsiloxane type cyclopolymers;
- mixtures of cyclic silicones with organic silicon derivatives;
- linear volatile silicones having two to nine silicon atoms and a viscosity less than or equal to $5.10^{-6} m^2/s$ at 25 °C.

**5.** Composition according to claim 1 or 2 characterised in that the non volatile silicones are selected from:

(a) polyalkylsiloxanes selected from:

- linear polydimethylsiloxanes having trimethylsilyl terminal groups and a viscosity of between $5.10^{-6}$ and 2.5 $m^2/s$ at 25 °C;
- linear polydimethylsiloxanes with dimethylsilanol terminal groups;
- poly ($C_1$-$C_{20}$)alkylsiloxanes;

(b) polyalkylarylsiloxanes selected from:

- polydimethylmethylphenylsiloxanes, polymethylphenylsiloxanes and linear or branched polydimethyldiphenylsiloxanes having a viscosity of between $1.10^{-5}$ and $5.10^{-2}$ $m^2/s$ at 25 °C;

(c) silicone gums, selected from polydiorganosiloxanes having molecular weights of between 200 000 and 1 000 000 used alone or mixed with a solvent;

(d) silicone resins containing the following units:

$R'_2SiO_{2/2}$, $R'SiO_{3/2}$, $SiO_{4/2}$

wherein R' represents a hydrocarbon group having one to six carbon atoms or a phenyl group;

(e) organomodified silicones selected from silicones comprising one or more organofunctional groups fixed to the siloxane chain either directly or via a hydrocarbon radical.

**6.** Composition according to claim 5 characterised in that the silicone gums used either alone or in a mixture are selected from the following:

- poly[(dimethylsiloxane)/(methylvinylsiloxane)],
- poly[(dimethylsiloxane)/(diphenylsiloxane)],
- poly[(dimethylsiloxane)/(phenylmethylsiloxane)],
- poly[(dimethylsiloxane)/(diphenylsiloxane)/(methylvinylsiloxane)]

and the following mixtures:

- mixtures formed from a chain end hydroxylated polydimethylsiloxane and a cyclic polydimethylsiloxane;
- mixtures formed from a polydimethylsiloxane gum and a cyclic silicone; and
- mixtures of polydimethylsiloxanes having different viscosities.

**7.** Composition according to claim 5 characterised in that the organomodified silicones are selected from polyorganosiloxanes comprising the following:

a) polyethyleneoxy and/or polypropyleneoxy groups;
b) substituted or unsubstituted amine groups;
c) thiol groups;
d) carboxylate groups;
e) alkoxyl groups;
f) hydroxyalkyl groups having the following formula:

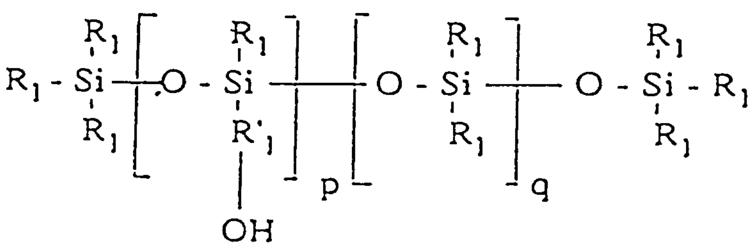

(I)

wherein:

radicals $R_1$, which may be identical or different, are selected from methyl and phenyl radicals, at least 60 mole % of radicals $R_1$ designating methyl;

radical $R'_1$ is a divalent hydrocarbonated $C_2$-$C_{18}$ alkylene link;

p is between 1 and 30 inclusive;

q is between 1 and 150 inclusive;

g) acyloxyalkyl groups having the following formula:

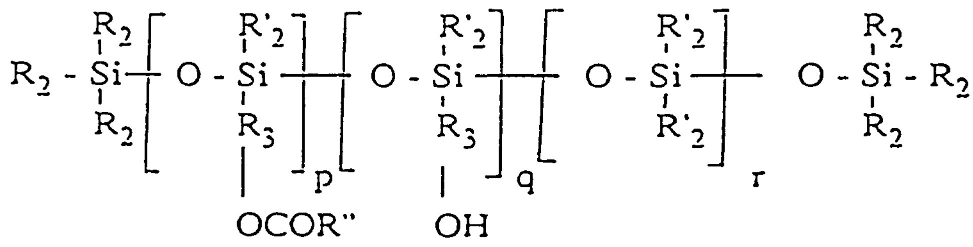 (II)

wherein:

$R_2$ represents a methyl, phenyl, -OCOR" or hydroxyl group where only one radical $R_2$ per silicon atom may be OH;

$R'_2$ represents methyl or phenyl, at least 60 mole % of the total of radicals $R_2$ and $R'_2$ representing methyl;

R" represents a $C_8$-$C_{20}$ alkyl or alkenyl;

$R_3$ represents a divalent hydrocarbonated alkylene linear or branched $C_2$-$C_{18}$ radical;

r is between 1 and 120 inclusive;

p is between 1 and 30;

q is zero or less than 0.5p, p + q being between 1 and 30;

polyorganosiloxanes of formula (II) possibly containing

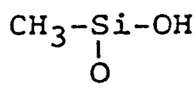

groups in proportions not exceeding 15 % of the sum p + q + r;

h) alkylcarboxyl groups;

i) 2-hydroxyalkylsulfonate groups;

j) 2-hydroxyalkylthiosulfate groups.

**8.** Composition according to claim 1 or 2 characterised in that the polyorganosiloxanes are selected from linear polyalkylsiloxanes with trimethylsilyl terminal groups and a viscosity of between 0.2 and 2.5 $m^2/s$ at 25 °C, mixtures of a chain end hydroxylated polydimethylsiloxane and a cyclic polydimethylsiloxane and mixtures of two polydimethylsiloxanes constituted by a gum and an oil having different viscosities.

**9.** Composition according to any one of claims 1 to 8 characterised in that the latex is a colloidal suspension of polymer particles obtained by polymerization or copolymerization of monomers selected from styrene, butadiene, acrylonitrile , chloroprene vinyl acetate, urethanes, isoprene, isobutylene and acrylic, methacrylic, maleic, crotonic or itacronic acid or their esters or amides.

**10.** Composition according to claim 9 characterised in that the latex which is insoluble in the aqueous medium is selected from homopolymers and copolymers of vinyl acetate, homopolymers and copolymers of acrylic acid, polyurethanes, carboxylated or non carboxylated butadiene/styrene copolymers, carboxylated or non carboxylated butadiene/acrylonitrile copolymers and styrene/acrylic ester copolymers.

**11.** Composition according to any one of claims 1 to 10 characterised in that the suspension agent is selected from compounds having formula:

a) $R_4X$ (III)

wherein $R_4$ is a long carbon chain aliphatic radical which may include oxygen atoms and X is a carboxylic, sulfuric or phosphoric acid residue or a radical derived from a carboxylic acid or an amide; these compounds of formula (III) are selected from those wherein:

(i) $R_4$ is a $C_{11}$-$C_{21}$ alkyl or alkenyl radical, X is:

. a COOA group wherein A is a mono- or polyhydroxyalkyl $C_2$-$C_3$ polyol derivative or a $CH_2CH_2SO_3M$ radical,

. a $CO(OCH_2CH_2)_n$-OH group where n is between 2 and 150,

. a

$$COOCH_2-\underset{\displaystyle CH_3}{\underset{|}{CH}}-(OCH_2CH_2)_nOH$$

group wherein n is between 2 and 150, the free OH groups of the groups defined above possibly being esterified using an acid $R_4COOH$ where $R_4$ is a $C_{11}$-$C_{21}$ alkyl or alkenyl group,

. a $CONR_5R_6$ group where $R_5$ or $R_6$ represent hydrogen or $C_1$-$C_4$ hydroxyalkyl, one at least representing $C_1$-$C_4$ hydroxyalkyl,

. a $OSO_3M$ or 1/3 $PO_4{}^{3\ominus}M_3$ group where M represents an alkali metal, ammonium or a $C_1$-$C_4$ alkanolamine residue;

(ii) $R_4$ represents a $R_7(OC_2H_4)_lOCH_2$ radical and X represents a COOM group where M has the meaning given above or $R_7$ represents a $C_{12}$-$C_{14}$ alkyl radical and l a whole or decimal number between 2.5 and 10 or $R_7$ represents oleyl and l is between 2 and 9 or $R_7$ represents $(C_8$-$C_9)$-alkylphenyl and l is between 4 and 8 or derivatives wherein $R_4$ represents a $(C_{12}$-$C_{16})$alkyl ether and X represents a $CONR_5R_6$ group wherein $R_5$ and $R_6$ have the meanings given above;

b) oxides of dimethyl $(C_{16}$-$C_{22})$alkylamines.

**12.** Composition according to any one of claims 1 to 11 characterised in that the suspension agent is an alcohol having 27 to 44 carbon atoms and including one or two ether and/or thioether or sulfoxide groups and has the formula (IV):

$R_8$ - X-$[C_2H_3(OH)]$-$CH_2$ - Y - $R_9$ (IV)

wherein:

$R_8$ and $R_9$, which may be identical or different, represent linear $C_{12}$-$C_{20}$ alkyl groups;

X represents an oxygen atom, a sulfur atom or a sulfoxide group;

Y represents an oxygen atom, a sulfur atom, a sulfoxide group or a methylene group; when Y represents a methylene group the sum of the number of carbon atoms in $R_8$ and $R_9$ is from 24 to 40, preferably from 26 to 36 inclusive, and when Y does not represent a methylene group the sum of the carbon atoms in $R_8$ and $R_9$ is from 24 to 40 inclusive, preferably from 28 to 36 inclusive; when X or Y represents sulfoxide, Y or X does not designate sulfur.

**13.** Composition according to any one of claims 1 to 12 characterised in that the composition comprises a mixture of a chain end hydroxylated polydimethylsiloxane and a cyclic polydimethylsiloxane, a latex which is a homopolymer or copolymer derived from acrylic acid and a thickening agent which is a crosslinked ammonium acrylate and acrylamide copolymer.

**14.** Composition according to any one of claims 1 to 13 characterised in that the silicone(s) are present in proportions of between 0.1 and 50% by weight, preferably between 0.2 and 30% by weight with respect to the total composition weight.

**15.** Composition according to any one of claims 1 to 14 characterised in that the latex is present in the composition in proportions of between 0.1 and 10% by weight, preferably between 0.5 and 5% by weight with respect to the total composition weight.

**16.** Composition according to any one of claims 1 to 15 characterised in that the thickening agent and/or suspension agent are present in the composition in proportions of between 0.1 and 20% by weight, preferably between 0.3 and 10% by weight with respect to the total composition weight.

**17.** Composition according to any one of claims 1 to 16 characterised in that it is in the form of a shampoo containing at least one surfactant detergent selected from anionic, non-ionic, amphoteric or zwitterionic

surfactants or mixtures thereof.

**18.** Method for a cosmetic treatment of keratinous material characterised in that a least one composition as defined in any one of claims 1 to 17 is applied to the keratinous material.

**19.** Use of a composition as defined in claim 17 as a shampoo.

**20.** Use of a composition as defined in any one of claims 1 to 16 as a mascara.

## Patentansprüche

**1.** Zusammensetzung zur Behandlung keratinischer Materien,
dadurch **gekennzeichnet**, daß
sie in einem wässrigen Milieu mindestens ein Silikon, mindestens einen in dem wässrigen Milieu unlöslichen Latex und mindestens ein Suspendiermittel für das Silikon und den Latex und/oder mindestens ein Verdickungsmittel enthält, ausgewählt aus Guar-Gummiprodukten, Gummi arabicum, Skleroglucanen, vernetzten oder nicht vernetzten Polyacrylsäuren, wässrigen Dispersionen von vernetzten Copolymeren aus Ammoniumacrylat/Acrylamid und aus der Emulsion des Copolymers von Acrylamid/Acrylamid von neutralisierter 2-Methylpropansulfonsäure.

**2.** Zusammensetzung gemäß Anspruch 1,
dadurch **gekennzeichnet**, daß
das Silikon aus in dem wässrigen Milieu unlöslichen oder löslichen Polyorganosiloxanen ausgewählt ist und in Form eines Öls, Wachses, Gummi- oder Harz-Produkts vorliegt.

**3.** Zusammensetzung gemäß Anspruch 2,
dadurch **gekennzeichnet**, daß
die Polysiloxane aus flüchtigen Silikonen mit einem Siedepunkt von 60 bis 260 °C ausgewählt sind.

**4.** Zusammensetzung gemäß Anspruch 3,
dadurch **gekennzeichnet**, daß
die flüchtigen Silikone ausgewählt sind aus:
- zyklischen Silikonen mit 3 bis 7 Siliziumatomen;
- Cyclopolymeren des Typs Dimethylsiolxan/Methylalkylsiloxan;
- Mischungen von zyklischen Silikonen mit organischen Siliziumderivatverbindungen;
- flüchtigen linearen Silikonen mit 2 bis 9 Siliziumatomen und einer Viskosität von weniger oder gleich $5 \times 10^{-6}$ $m^2/s$ bei 25 °C.

**5.** Zusammensetzung gemäß Anspruch 1 oder 2,
dadurch **gekennzeichnet**, daß
die nicht flüchtigen Silikone ausgewählt sind aus:
(a) Polyalkylsiloxanen, ausgewählt aus:
- linearen Polydimethylsiloxanen mit endständigen Trimethylsilylgruppen einer Viskosität von $5 \times 10^{-6}$ bis 2,5 $m^2/s$ bei 25 °C;
- linearen Polydimethylsiloxanen mit endständigen Dimethylsilanolgruppen;
- Poly($C_{1-20}$)alkylsiloxanen;

(b) Polyalkylarylsiloxanen, ausgewählt aus:
- linearen und/oder verzweigten Polydimethylmethylphenylsiloxanen, Polymethylphenylsiloxanen, Polydimethyldiphenylsiloxanen einer Viskosität von $1 \times 10^{-5}$ bis $5 \times 10^{-2}$ $m^2/s$ bei 25 °C;

(c) Silikon-Gummiprodukten, ausgewählt aus Polydiorganosiloxanen mit Molekularmassen von 200000 bis 1000000, die alleine oder in Form einer Mischung in einem Lösungsmittel verwendet werden;
(d) Silikon-Harzen, aufgebaut aus Einheiten:
$R'_2SiO_{2/2}$, $R'SiO_{3/2}$, $SiO_{4/2}$
in denen R' eine Kohlenwasserstoffgruppe mit 1 bis 6 Kohlenstoffatomen oder eine Phenylgruppe darstellt;
(e) organomodifizierten Silikonen, ausgewählt aus Silikonen, die in ihrer Struktur eine oder mehrere organofunktionelle Gruppen aufweisen, die direkt oder über das Zwischenglied eines Kohlenwasserstoffrestes an die Siloxankette gebunden sind.

**6.** Zusammensetzung gemäß Anspruch 5, dadurch **gekennzeichnet**, daß die Silikon-Gummiprodukte, die alleine oder in Form einer Mischung verwendet werden, aus den folgenden Strukturen:

- Poly((dimethylsiloxan)/(methylvinylsiloxan)),
- Poly((dimethylsiloxan)/(diphenylsiloxan)),
- Poly((dimethylsiloxan)/(phenylmethylsiloxan)),
- Poly((dimethylsiloxan)/(diphenylsiloxan)/(methylvinylsiloxan))

und aus den folgenden Mischungen ausgewählt sind:

- Mischungen, die aus einem am Kettenende hydroxylierten Polydimethylsiloxan und einem zyklischen Polydimethylsiloxan gebildet sind
- Mischungen, die aus einem Polydimethylsiloxan-Gummi und einem zyklischen Silikon gebildet sind und
- Mischungen aus Polydimethylsiloxanen unterschiedlicher Viskositäten.

**7.** Zusammensetzung gemäß Anspruch 5, dadurch **gekennzeichnet**, daß die organomodifizierten Silikone aus Polyorganosiloxanen ausgewählt sind, die aufweisen:

a) Polyethylenoxy- und/oder Polypropylenoxy-Gruppen;
b) substituierte oder nicht substituierte Amingruppen;
c) Thiolgruppen;
d) Carboxylatgruppen;
e) Alkoxygruppen;
f) Hydroxyalkylgruppen der folgenden Formel:

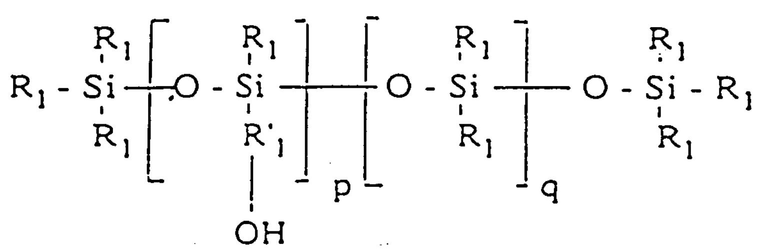

(I)

worin gilt:
die Reste $R_1$ welche gleich oder verschieden sind, sind aus Methyl- und Phenylresten ausgewählt, wobei mindestens 60 Mol% der Reste $R_1$ den Methylrest darstellen;
der Rest $R'_1$ ist ein zweiwertiges $C_{2-18}$-Alkylen-Kettenglied;
p beträgt 1 bis 30;
q beträgt 1 bis 150;
g) Acyloxyalkylgruppen der folgenden Formel:

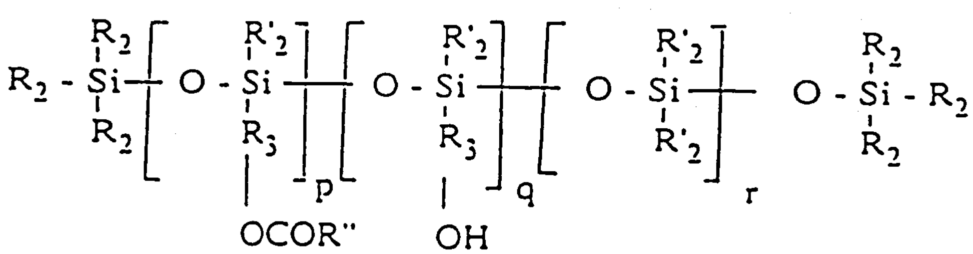

(II)

worin gilt:
$R_2$ bedeutet eine Methyl-, Phenyl-, -OCOR''-, Hydroxylgruppe, wobei ein einziger der Reste $R_2$ pro Siliziumatom eine OH-Gruppe sein kann;

$R'_2$ bedeutet einen Methyl- oder Phenylrest, wobei mindestens 60 Mol% der Gesamtheit der Reste $R_2$ und $R'_2$ den Methylrest darstellen;
R'' bedeutet einen $C_{8-20}$-Alkyl- oder -Alkenylrest;
$R_3$ bedeutet einen zweiwertigen, linearen oder verzweigten $C_{2-8}$-Alkylenrest;
r beträgt 1 bis 120;
p beträgt 1 bis 30;
q ist gleich 0 oder beträgt weniger als 0,5 p, wobei p + q 1 bis 30 beträgt; die Polyorganosiloxane der Formel (II) können

$CH_3$-Si-OH-Gruppen
|
O
|

in Mengenanteilen enthalten, die 15% der Summe p + q + r nicht überschreiten;
h) Alkylcarboxylgruppen;
i) 2-Hydroxyalkylsulfonatgruppen;
j) 2-Hydroxyalkylthiosulfatgruppen.

**8.** Zusammensetzung gemäß Anspruch 1 oder 2,
dadurch **gekennzeichnet**, daß
die Polyorganosiloxane aus linearen Polyalkylsiloxanen mit endständigen Trimethylsilylgruppen einer Viskosität von 0,2 bis 2,5 $m^2/s$ bei 25 °C, aus Mischungen eines am Kettenende hydroxylierten Polydimethylsiloxans und eines zyklischen Polydimethylsiloxans sowie aus Mischungen von zwei Polydimethylsiloxanen ausgewählt sind, die aus einem Gummiprodukt und einem Öl mit unterschiedlichen Viskositäten zusammengesetzt sind.

**9.** Zusammensetzung gemäß jedem der Ansprüche 1 bis 8,
dadurch **gekennzeichnet**, daß
der Latex, der eine kolloidale Suspension von Polymerpartikeln ist, durch Polymerisation, Copolymerisation von Monomeren erhältlich ist, ausgewählt aus Styrol, Butadien, Acrylnitril, Chloropren, Vinylacetat, Urethanen, Isopren, Isobutylen, Acryl-, Methacryl-, Malein-, Croton-, Itaconsäuren oder aus deren Estern oder deren Amiden.

**10.** Zusammensetzung gemäß Anspruch 9,
dadurch **gekennzeichnet**, daß
der in dem wässrigen Milieu unlösliche Latex aus Homopolymeren und Copolymeren von Vinylacetat, Homopolymeren und Copolymeren von Acrylsäure, Polyurethanen, Butadien/Styrol-Copolymeren, die carboxyliert sind oder nicht, aus Butadien/Acrylnitril-Copolymeren, die carboxyliert sind oder nicht, sowie aus Styrol/Acrylester-Copolymeren ausgewählt ist.

**11.** Zusammensetzung gemäß jedem der Ansprüche 1 bis 10,
dadurch **gekennzeichnet**, daß
das Suspendiermittel aus Verbindungen der Formel ausgewählt ist:
a) $R_4X$ (III),
worin $R_4$ ein aliphatischer Rest mit langer Kohlenstoffkette, die gegebenenfalls durch Sauerstoffatome unterbrochen ist, und X ein Carboxyl-, Schwefel- oder Phosphorsäurerest oder ein von einer Carboxylsäure oder einem Amid abgeleiteter Rest sind; diese Verbindungen der Formel (III) sind aus denjenigen ausgewählt, in denen gilt:
(i) $R_4$ ist ein $C_{11-21}$-Alkyl oder -Alkenylrest und X ist:
- eine COOA-Gruppe, worin A ein Mono- oder Polyhydroxyalkylrest, der von einem $C_{2-3}$-Polyol abgeleitet ist, oder ein $-CH_2CH_2SO_3M$-Rest ist,
- eine $-CO(OCH_2CH_2)_n-OH$-Gruppe, worin n einen Wert von 2 bis 150 besitzt,

- eine

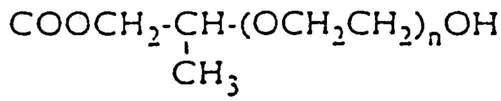

-Gruppe,
worin n einen Wert von 2 bis 150 aufweist, und wobei die OH-Funktonen, die von den oben definierten Gruppen frei sind, mit einer Säure $R_4COOH$ verestert sein können, worin $R_4$ ein $C_{11-21}$-Alkyl- oder -Alkenylrest ist,
- eine $CONR_5R_6$-Gruppe, worin $R_5$ und $R_6$ Wasserstoff oder einen $C_{1-4}$-Hydroxyalkylrest darstellen, wobei einer mindestens einen $C_{1-4}$-Hydroxyalkylrest darstellt,
- eine $OSO_3M$- oder 1/3 $PO_4{}^{3-}M_3$-Gruppe, worin M ein Alkalimetall, den Ammonium- oder einen $C_{1-4}$-Alkanolaminrest darstellt;

(ii) $R_4$ bedeutet einen $R_7(OC_2H_4)_lOCH_2$-Rest und X bedeutet eine COOM-Gruppe, worin M die oben angegebene Bedeutung hat, und wobei $R_7$ einen $C_{12-14}$-Alkylrest darstellt und l eine ganze Zahl oder eine Dezimalzahl von 2,5 bis 10 ist, oder $R_7$ bedeutet auch den Oleyl-Rest und l beträgt 2 bis 9, oder $R_7$ bedeutet auch einen $C_{8-9}$-Alkylphenylrest und l beträgt 4 bis 8, oder es handelt sich um Derivate, in denen $R_4$ eine $C_{12-16}$-Alkylethergruppe und X eine $-CONR_5R_6$-Gruppe bedeuten, in denen $R_5$ und $R_6$ die oben bereits angegebene Bedeutung haben;

b) aus Dimethyl($C_{16-22}$)alkylaminoxiden.

**12.** Zusammensetzung gemäß jedem der Ansprüche 1 bis 11,
dadurch **gekennzeichnet**, daß
das Suspendiermittel ein Alkohol mit 27 bis 44 Kohlenstoffatomen ist und eine oder zwei Ether- und/oder Thioether- oder Sulfoxidgruppen enthält, gemäß der Formel (IV):

$R_8 - X - (C_2H_3(OH))-CH_2 - Y - R_9$ (IV)

worin gilt:
$R_8$ und $R_9$ bedeuten, gleich oder verschieden, lineare $C_{12-20}$-Alkylgruppen;
X bedeutet ein Sauerstoffatom, Schwefelatom oder eine Sulfoxidgruppe;
Y bedeutet ein Sauerstoffatom, Schwefelatom, eine Sulfoxid- oder Methylengruppe;
wenn Y eine Methylengruppe bedeutet, beträgt die Summe der Anzahl der Kohlenstoffatome von $R_8$ bis $R_9$ 24 bis 40, vorzugsweise 26 bis 36, und wenn Y keine Methylengruppe bedeutet, beträgt die Summe der Kohlenstoffatome von $R_8$ und $R_9$ 24 bis 40, vorzugsweise 28 bis 36; wenn X oder Y die Sulfoxidgruppe bedeuteten, stellen Y oder X kein Schwefelatom dar.

**13.** Zusmamensetzung gemäß jedem der Ansprüche 1 bis 12,
dadurch **gekennzeichnet**, daß
die Zusammensetzung eine Mischung aus einem am Kettenende hydroxylierten Polydimethylsiloxan und einem zyklischen Polydimethylsiloxan, Latex, der ein von Acrylsäure abgeleitetes Homopolymer oder Copolymer ist, sowie Verdickungsmittel enthält, das ein vernetztes Copolymer von Ammoniumacrylat und Acrylamid ist.

**14.** Zusammensetzung gemäß jedem der Ansprüche 1 bis 13,
dadurch **gekennzeichnet**, daß
das oder die Silikone in Mengenanteilen von 0,1 bis 50, vorzugsweise von 0,2 bis 30, Gew.% vorhanden sind, bezogen auf das Gesamtgewicht der Zusammensetzung.

**15.** Zusammensetzung gemäß jedem der Ansprüche 1 bis 14,
dadurch **gekennzeichnet**, daß
der Latex in der Zusammensetzung in Mengenanteilen von 0,1 bis 10, vorzugsweise von 0,5 bis 5, Gew.% vorliegt, bezogen auf das Gesamtgewicht der Zusammensetzung.

**16.** Zusammensetzung gemäß jedem der Ansprüche 1 bis 15,
dadurch **gekennzeichnet**, daß
das Verdickungsmittel und/oder Suspendiermittel in den Zusammensetzungen in Mengenanteilen von 0,1 bis 20, vorzugsweise von 0,3 bis 10, Gew.% vorhanden sind, bezogen auf das Gesamtgewicht der Zusammensetzung.

**17.** Zusammensetzung gemäß jedem der Ansprüche 1 bis 16,
dadurch **gekennzeichnet**, daß
sie in Form eines Shampoo vorliegt, das zusätzlich mindestens ein oberflächenaktives Detergens enthält, ausgewählt aus anionischen, nicht-ionischen, amphoteren oder zwitterionischen oberflächenaktiven Mitteln oder aus deren Mischungen.

**18.** Verfahren zur kosmetischen Behandlung keratinischer Materien,
dadurch **gekennzeichnet**, daß
man auf diese Materien mindestens eine in jedem der Ansprüche 1 bis 17 definierte Zusammensetzung aufträgt.

**19.** Verwendung einer in Anspruch 17 definierten Zusammensetzung als Shampoo.

**20.** Verwendung einer in jedem der Ansprüche 1 bis 16 definierten Zusammensetzung als Schmink-Maske.